# EUROPEAN PATENT APPLICATION

(11) **EP 1 129 710 A2**
(43) Date of publication of application: **05.09.2001**
(21) Application number: 00310391.8
(22) Date of filing: 23.11.2000
(51) Int. Cl.: A61K 31/192, A61P 25/06

(54) **Method for treating migraine symptoms with ibuprofen and salts thereof**

(30) Priority: 24.11.1999 US 449124; 09.11.2000 US 709069
(71) Applicant: McNEIL-PPC, Inc., Skillman, New Jersey 08558 (US)
(72) Inventor: Codispoti, Joseph R., Philadelphia, PA 19116 (US)
(74) Representative: Mercer, Christopher Paul

(57) **Abstract**

The present invention provides a method for treating the photophobia and phonophobia symptoms of migraine attacks with an effective amount of ibuprofen, pharmaceutically acceptable salts thereof, isomers thereof, or mixtures thereof.

## Description

### Field of the Invention

The present invention relates to the treatment of migraine symptoms by the administration of an effective amount of ibuprofen, more specifically the present invention relates to the treatment of phonophobia and photophobia with ibuprofen.

### Background of the Invention

Migraine provides a wide variation of pain and symptoms, and associated disorders with those who suffer from the disease. Included among these symptoms are nausea, headache, moderate to severe pain, as well as incapacitating pain and total disability in a percentage of patients. Some patients also recite more specific symptoms such as photophobia, painful sensitivity to light; or phonophobia, painful sensitivity to sound.

Treatment of migraine pain includes both prescription and non-prescription or over-the-counter medication. Among the over-counter medication that is currently available is a combination of acetaminophen/aspirin/caffeine, as set forth in US Patent 5,972,916, the contents hereby incorporated by reference as set forth herein in its entirety. The combination of active ingredients suffers from the drawback that some of the actives might cause undesired side effects. For example, it is well know that aspirin can cause stomach irritation and caffeine can cause anxiety and sleeplessness.

Therefore there is a continuing need for treatment of migraine, as well as treatment alternatives to specific migraine symptoms.

### Summary of the Invention

The present invention provides a method for treating photophobia and phonophobia associated with a migraine attack by providing an effective amount of ibuprofen, pharmaceutically acceptable salts thereof, isomers thereof, or mixtures thereof. The present invention relies on the action of a single active ingredient, i.e., ibuprofen, pharmaceutically acceptable salts thereof, isomers thereof, or mixtures thereof. The present invention also contemplates the use of additional ingredients, such as flavors, binders and excipients.

### Description of the Drawings

Figure 1 depicts the percentage of subjects with reduced photophobia over time after treatment with ibuprofen.

Figure 2 depicts the percentage of subjects with reduced phonophobia over time over time after treatment with ibuprofen.

### Detailed Description of the Invention

Ibuprofen is a well known analgesic material that has analgesic and antipyrritic properties. It is commercially available in various forms for many years.

According to the present invention, ibuprofen, pharmaceutically acceptable salts thereof, isomers thereof, or mixtures thereof, are preferably administered as an oral dose, as an effective dose to alleviate the photophobia and phonophobia symptoms, which comprises from about 100 to about 800 milligrams of ibuprofen per dose; preferably from 200 to about 600 and most preferably from about 300 to about 400 milligrams of ibuprofen per dose. Typical doses of the pharmaceutically acceptable salts of ibuprofen will vary according to the molecular weight of the salt required to give the equivalent dose of ibuprofen. Typical oral doses of pharmaceutically acceptable salts of ibuprofen range from about 110 to about 1700 milligrams per dose. Typical doeses of mixtures of ibuprofen or its isomers with pharmaceutically acceptable salts thereof range from about 100 to about 1700 milligrams per dose, preferably from about 200 to about 1300 milligrams per dose, and most preferably from about 300 to about 850 milligrams per dose. Typically doses are taken every four to six hours, but care should be taken to avoid exceeding the daily maximum recommended dosage of ibuprofen of 2400 milligrams per day.

Examples of suitable pharmaceutically acceptable salts of ibuprofen include any of the inorganic cation salts such as sodium, potassium, lithium, magnesium, calcium, cesium, ammonia, ferrous, zinc, manganous, aluminum, ferric, and manganic; organic salts of ibuprofen with primary, secondary, tertiary and quaternary amines, or mixtures thereof. Examples of such primary, secondary, tertiary and quaternary amines include substituted amines including but not limited to naturally occurring substituted amines, cyclic amines, basic ion exchange resins, and mixtures thereof. More specifically, suitable amines include but are not limited to triethylamine, tripropylamine, 2-dimethylaminoethanol, 2-diethylaminoethanol, lysine, arginine, histidine, caffeine, procain, N-ethylpiperidine, hydrabamine, choline, betaine, ethylenediamine, glucosamine, TRIS(hydroxymethyl)-aminomethane, methylglycamine, theobromine, pruines, piperazine, piperidine, polyamine resins and the like, and mixtures thereof. In one embodiment, a mixture of ibuprofen and its sodium salt may be used.

Examples of suitable isomers include, but are not limited to R-ibuprofen, S-ibuprofen and mixtures thereof.

Suitable dosage forms include solids or liquids. Solid forms include tablets, capsules, liquid-filled soft gelatin capsules, powders, sachets and the like. Suitable liquids include suspensions, solutions, emulsions and the like.

The ibuprofen, pharmaceutically acceptable salts thereof, isomers thereof, or mixtures thereof, may be admixed with various pharmaceutically-acceptable excipients and other ingredients including but not limited to diluents, granulating agents, disintegrating agents, binding agents, lubricants and the like. Examples of these various ingredients are set forth in US Patent 5,660,860, the contents hereby incorporated by reference as if set forth in its entirety.

The present invention as set forth in the following examples are meant to exemplify the various aspects of carrying out the present invention and are not intended to limit the invention in any way.

### Example 1

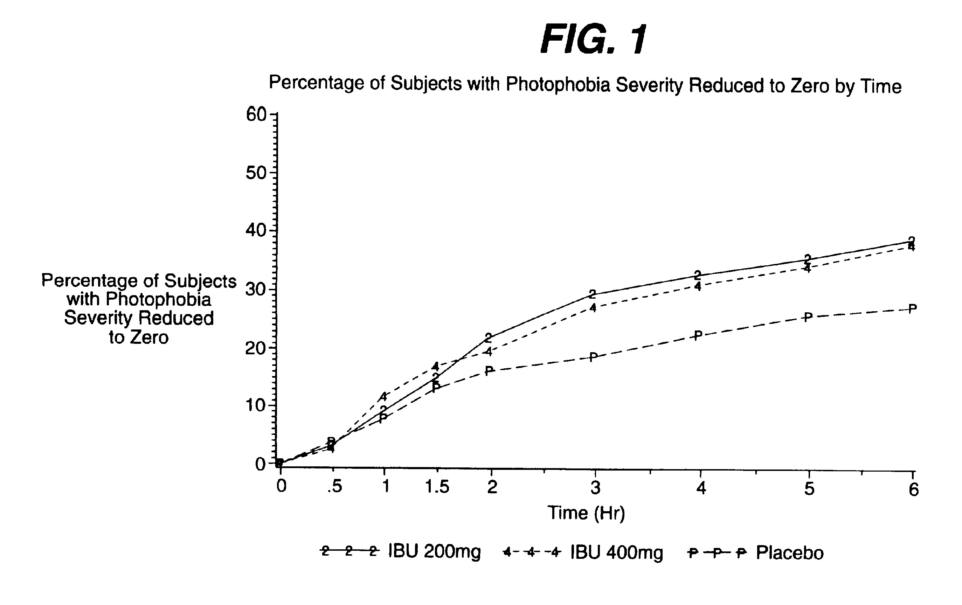

Subjects suffering from photophobia due to a migraine were either given a placebo or treated with 200 or 400 milligrams of ibuprofen. Approximately 650 subjects were in the study. Some subjects were given placebos, while others were given either 200 or 400 milligrams of ibuprofen. Periodic assessments of their photophobia were made and the results are presented in Figure 1. The results indicate that at time of two hours or longer the treatment of photophobia with ibuprofen was effective in relieving photophobia

### Example 2

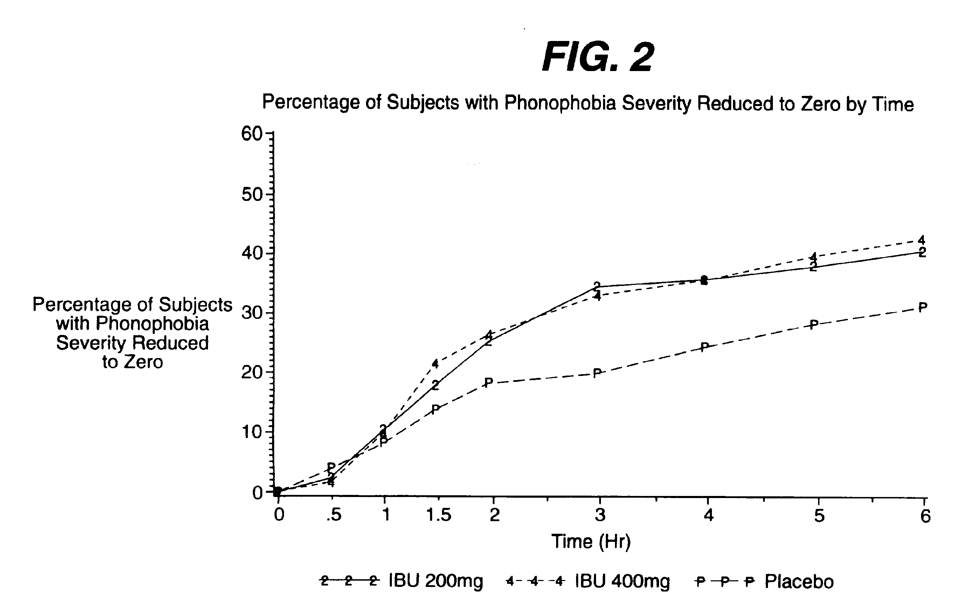

Subjects suffering from phonophobia due to a migraine were either given a placebo or treated with 200 or 400 milligrams of ibuprofen in tablet form. Periodic assessments of their photophobia were made and the results are presented in Figure 2. The results indicate that at time of two hours or longer the treatment of photophobia with ibuprofen was effective in relieving photophobia.

## Claims

1. A composition comprising ibuprofen, a pharmaceutically acceptable salt thereof, an isomer thereof or a mixture thereof, as the sole pharmaceutically effective ingredients, for mitigating or treating photophobia or phonophobia associated with migraine.

2. The composition of claim 1 wherein the amount of ibuprofen, isomer thereof or mixture thereof, is from 100 to 800 milligrams, preferably from 200 to 600 milligrams, per dose.

3. The composition of claim 2 wherein the amount of ibuprofen, isomer thereof or mixture thereof, is about 200 milligrams per dose.

4. The composition of claim 1 wherein the amount of ibuprofen isomer thereof or mixture thereof, is about 400 milligrams per dose.

5. The composition of claim 1 wherein the amount of the mixture of ibuprofen, an isomer thereof or a pharmaceutically acceptable salt thereof is from 100 to 1700 milligrams per dose, preferably from 200 to 1300 milligrams per dose.

6. The composition of any one of claims 1 to 5 wherein the pharmaceutically acceptable salt of ibuprofen is:
a) an inorganic cation salt;
b) an organic salt of ibuprofen with a pharmaceutically acceptable primary, secondary, tertiary or quaternary amine; or
c) a mixture thereof.

7. The composition of claim 6 wherein the pharmaceutically acceptable salt of ibuprofen is:
a) an inorganic sodium, potassium, lithium, magnesium, calcium, cesium, ammonium, ferrous, zinc, manganous, aluminum, ferric or manganic cation salt; or
b) a triethylamine, tripropylamine, 2-dimethylaminoethanol, 2-diethylaminoethanol, lysine, arginine, histidine, caffeine, procain, N-ethylpiperidine, hydrabamine, choline, betaine, ethylenediamine, glucosamine, TRIS(hydroxymethyl)aminomethane, methylglycamine, theobromine, pruine, piperazine, piperidine or polyamine resin organic salt; or
c) a mixture thereof.

8. The composition of any one of claims 1 to 7 wherein the mixture is a mixture of ibuprofen and its sodium salt.

9. The composition of any one of claims 1 to 8 wherein the isomer of ibuprofen is R-ibuprofen, S-ibuprofen or a mixture thereof.
